# EUROPÄISCHE PATENTSCHRIFT

(11) **EP 3 199 187 B1**
(45) Veröffentlichungstag und Bekanntmachung des Hinweises auf die Patenterteilung: **12.12.2018**
(21) Anmeldenummer: 17150609.0
(22) Anmeldetag: 09.01.2017
(51) Int. Cl.: A61M 5/00, B65D 25/10, B01L 9/00

(54) **TRÄGERPLATTE FÜR PHARMAZEUTISCHE BEHÄLTNISSE**
HOLDER PLATE FOR PHARMACEUTICAL CONTAINERS
PLAQUE DE SUPPORT POUR RÉCIPIENTS PHARMACEUTIQUES

(30) Priorität: 28.01.2016 DE 102016201268
(43) Veröffentlichungstag der Anmeldung: 02.08.2017
(73) Patentinhaber: SCHOTT Schweiz AG, 9001 St. Gallen (CH)
(72) Erfinder: Auerbach, Judith, 9052 Niederteufen (CH)
(74) Vertreter: Sawodny, Michael-Wolfgang

(56) Entgegenhaltungen:
- EP-A1- 1 138 390
- WO-A1-2012/126582
- US-A1- 2015 041 349

## Beschreibung

Die Erfindung, so wie in den Ansprüchen definiert, betrifft eine Trägerplatte für pharmazeutische Behältnisse oder medinzinsche Packmittel, insbesondere Spritzen, Fläschchen oder Karpulen, umfassend eine Mehrzahl von Durchgangsöffnungen zur Aufnahme von pharmazeutischen Behältnissen oder medizinischen Packmitteln. Die Trägerplatte weist des Weiteren Erhebungen auf.

Des Weiteren betrifft die Erfindung eine Transport- und/oder Lagereinrichtung für pharmazeutische Behältnisse oder medizinische Packmittel.

Zur effektiven Herstellung pharmazeutischer Behältnisse oder medizinischer Packmittel, insbesondere Spritzen, Fläschchen oder Karpulen werden diese in vordefinierten Anordnungen, sogenannten Nestern, vorkonfektioniert, in ihrer Lage zueinander definiert gehalten und so den jeweils erforderlichen Verarbeitungsprozessen nacheinander und/oder gleichzeitig ausgesetzt. Der Transport und die Lagerung der pharmazeutischen Behältnisse oder medizinischen Packmittel von/zu den und innerhalb der einzelnen, die Verarbeitungsprozesse ausführenden Vorrichtungen sowie die Positionierung innerhalb dieser Vorrichtungen erfolgt ebenfalls in diesen vordefinierten Anordnungen. Dazu werden die pharmazeutischen Behältnisse oder medizinischen Packmittel in einer Aufnahmeeinrichtung in Form einer Trägerplatte in ihrer Lage zueinander und gegenüber der Trägerplatte definiert gehalten und gelagert. Dadurch wird die gleichzeitige Herstellung einer hohen Anzahl derartiger pharmazeutischer Behältnisse oder medizinischer Packmittel in einem Verarbeitungsprozessschritt sichergestellt. Die pharmazeutischen Behältnisse oder medizinischen Packmittel werden im einzelnen in Aufnahmeöffnungen, insbesondere Durchgangsöffnungen enthaltenden Trägerplatten eingehängt, mit diesen verklemmt oder anderweitig an diesen positioniert, um die Behältnisse oder Packmittel beim Transport vor Beschädigungen zu schützen und eine gleichzeitige Weiterverarbeitung der gesamten Behältnisanordnung zu gewährleisten.

Insbesondere dienen derartige vordefinierte Anordnungen mit zentrierter Führung der einzelnen Behältnisse oder Packmittel in den jeweiligen Durchgangsöffnungen der Vereinfachung der anordnungsweisen gemeinsamen Weiterverarbeitung in vordefinierten Verfahrensschritten, beispielsweise der Sterilisation der Behältnisse, dem Befüllen der Behältnisse, einen gemeinsamen sicheren Transport zu und von den jeweiligen Verarbeitungsvorrichtungen, dem Verschließen der Behältnisse, usw. Eine gemeinsame Weiterverarbeitung einer derartigen Anordnung, insbesondere ein Befüllen und Verschließen der pharmazeutischen Behältnisse oder Packmittel kann beispielsweise in einer Vorrichtung, wie in der WO 2011/000606 A1 beschrieben, erfolgen, indem die Anordnung der pharmazeutischen Behältnisse durch eine Trägerplatte fixiert den einzelnen Verfahrensschritten unterzogen wird. Derartige Trägerplatten umfassen eine Mehrzahl von Durchgangsöffnungen zur Aufnahme der pharmazeutischen Behältnisse oder Packmittel aufweisenden Aufnahmebereich, einen definierten Handhabungsbereich zum Angriff an der Trägerplatte und/oder Lagerung dieser. Die Trägerplatte ist in der Regel aus Kunststoff gefertigt. Die Trägerplatte mit den in diesen positionierten und zentrierten pharmazeutischen Behältnissen oder Packmitteln ist bei den vorgenannten Verarbeitungs- und Transportprozessen jedoch einer Vielzahl von Belastungen ausgesetzt, die zu unerwünschten Verformungen an dieser führen und das Handling der Trägerplatte sowie die Qualität der Verarbeitungsprozesse erheblich beeinträchtigen. Auch aus der US 2009/100802 A1, , der WO 2011 /007194 A1 und der US 3643812 A sind unterschiedliche Transport- und/oder Lagereinrichtungen für pharmazeutische Behältnisse oder medizinische Packmittel, insbesondere Spritzen, Fläschchen und Kapulen bekannt geworden, bei denen eine Vielzahl der pharmazeutischen Behältnisse in einer vordefinierten Position lagerbar ist. Ein weiteres Problem aus dem Stand der Technik ist, dass während des Transportes oder auch in der Abfüllanlage durch eine weitere Handhabung der Trägerplatten mit eingesetzten pharmazeutischen Behältnissen oder medizinischen Packmitteln diese sich verdrehen können. In einem derartigen Fall sind die einzelnen pharmazeutischen Behältnisse oder medizinischen Packmittel nicht mehr ausgerichtet und liegen teilweise sogar übereinander. Insbesondere tritt dies auf, wenn die pharmazeutischen Behältnisse oder medizinschen Packmittel sich in axialer Richtung bewegen und es dabei zu einem Verdrehen kommt. Um ein Verdrehen zu vermeiden beschreibt beispielsweise die WO 2012/126582 eine Trägerplatte mit Einrichtungen zur Verdrehsicherung in Form von Stegen, die Anschlagabschnitte für einen Spritzenkragen aufweisen und an denen die Spritzenkragen der pharmazeutischen Behältnisse anliegen können. Aus der WO 2012/126582 ist aber nicht bekannt wie ein axiales Bewegen der Behältnisse, die anschließend zu einem Verdrehen führen, verhindert werden kann. Des Weiteren ist aus der WO 2012/126582 nicht bekannt, wie nach einem axialen Bewegen der pharmazeutischen Behältnisse diese wieder in die Öffnung eingefädelt werden können.

Ein axiales Bewegen wird beispielsweise durch eine Zusatzplatte, wie in der US 20130186793 A1 beschrieben oder durch Überstände wie in der EP 1 449 551 B1 gezeigt, vermieden.

Aus der US 2010/0059461 A1 ist eine Trägerplatte für pharmazeutische Erzeugnisse bekannt geworden, wobei die pharmazeutischen Erzeugnisse in Öffnungen der Trägerplatte eingesetzt und mittels Erhebungen in der Position gehalten werden. Die US 2010/0059461 A1 zeigte keine Erhebungen mit Schrägen und/oder Radien, die mit dem Flansch des Packmittels zusammenwirken, derart, dass die Packmittel ausgerichtet werden. Desweiteren dienen die aus der US 2010/0059461 A1 bekannten Erhebungen dazu die Packmittel zu verriegeln, nicht jedoch zu deren Ausrichtung. Die US 5,080,232 zeigt ebenfalls eine Trägerplatte mit Öffnungen, in die pharmazeutische Behälter eingesetzt werden können. Die Öffnungen sind mit Haltevorrichtungen versehen, um die eingesetzten pharmazeutischen Behälter in Position zu halten. Eine Ausrichtung oder Einfädeln der pharmazeutischen Erzeugnisse ist in der US 5, 080,232 nicht beschrieben.

Aus dem nächstliegenden Stand der Technik in Form der US 2015/0041349A1 ist eine Trägerplatte bekannt geworden, die eine Vielzahl von Durchgangsöffnungen mit Schrägen und Radien umfasst.

Die EP 1138390A1 zeigt eine Transportvorrichtung für medizinische Behälter, insbesondere Packmittel für medizinische Substanzen. Die Transportvorrichtung umfasst eine Trägerplatte mit einer Vielzahl von Öffnungen zur Aufnahme von Behältern, die einen Überstand aufweisen.

Die WO 2012/126582 zeigt eine Trägerplatte für pharmazeutische Behältnisse, die in Öffnungen der Trägerplatte eingesetzt werden.

Aufgabe der Erfindung ist es die Nachteile des Standes der Technik zu überwinden und insbesondere eine Trägerplatte anzugeben, bei der die pharmazeutischen Behältnisse oder medizinischen Packmittel mit wenigstens einem nicht symmetrischen Flansch auch nach einem Hochhüpfen, d.h. einer Bewegung in axialer Richtung, wieder in die Öffnung eingefädelt werden und/oder ein nachfolgendes Verdrehen der pharmazeutischen Behältnisse oder medizinischen Packmitteln wenigstens mit einem nicht zylindersymmetrischen Flanschen oder Kragen, vermieden wird. Gelöst wird die Aufgabe durch eine Trägerplatte gemäß Anspruch 1.

Die erfindungsgemäße Trägerplatte für pharmazeutische Behältnisse oder medizinische Packmittel mit einem nicht zylindersymmetrischen Flansch oder Kragen, insbesondere Spritzen, Fläschchen oder Karpulen, umfasst eine Mehrzahl von Durchgangsöffnungen zur Aufnahme der pharmazeutischen Erzeugnisse sowie eine über die Oberseite der Trägerplatte hinausragende Erhebung und/oder einzelstehenden Bauteilen mit einer Höhe und wenigstens einer Schräge und/oder einem Radius umfasst, wobei jeder der Durchgangsöffnungen wenigstens eine Erhebung und/oder ein Bauteil die bzw. das wenigstens eine Schräge und/oder Radius aufweist, zugeordnet sind. Die Schräge und/oder der Radius wirken mit dem nicht zylindersymmetrischen Flansch oder Kragen zusammen, derart, dass die pharmazeutischen Behältnisse oder medizinischen Packmittel ausgerichtet sind.

Unter Ausrichtung wird verstanden, dass die Schrägen/Radien das Einfädeln von in die Trägerplatte eingebrachten pharmazeutischen Behältnissen oder medizinischen Packmitteln erleichtern und eine Zentrierung der pharmazeutischen Behältnisse ermöglichen. Insbesondere sorgen die Schrägen/Radien auch dafür, dass sich die pharmazeutischen Behältnisse oder medizinischen Packmittel nicht verdrehen bzw. falls sie sich drehen, die pharmazeutischen Behältnisse oder medizinischen Packmittel durch die Schrägen und/oder Radien in die vorgegebene Position zurückverbracht werden. Die Schrägen/Radien dienen auch dazu, die pharmazeutischen Behältnisse nach einem Hochhüpfen wieder in die vorgegebene Position zu verbringen.

Die Wirkung einer Ausrichtung der pharmazeutischen Behältnisse tritt nur dann ein, wenn die pharmazeutischen Behältnisse nicht unter zu starker Fehlstellung eingesetzt werden. Des Weiteren erfolgt auch dann keine Ausrichtung, wenn die pharmazeutischen Behältnisse exakt richtig eingesetzt werden. Eine Selbstausrichtung der pharmazeutischen Behältnisse wird bevorzugt bei geringen Winkelabweichungen von Winkeln > 0° bis ungefähr 20° beobachtet.

In einer fortgebildeten Ausführungsform umfasst die Erhebung eine Höhe, und die Höhe der über die Oberseite der Trägerplatte hinausragende Erhebung beträgt mindestens 2 mm, bevorzugt 2 mm bis 20 mm, insbesondere 4 mm bis 10 mm.

Durch die über die Oberseite der Trägerplatte hinausragende Erhebung wird das pharmazeutische Behältnis zumindest immer im Rahmen der angegebenen Höhe der Erhebung gegen Heraushüpfen durch die axiale Höhe gesichert.

Die Höhe von mindestens 2 mm der Erhebungen und/oder einzelstehenden Bauteile in Verbindung mit den Schrägen und/oder Radien verhindern ein Verdrehen z. B. auch beim axialen Hochhüpfen. Im Gegensatz zur EP 1 449 551 B1 und US 20130186793 A1 sind hierfür keine zusätzlichen Platten oder Vorsprünge notwendig. Dadurch, dass ein Verdrehen der einzelnen pharmazeutischen Behältnisse mit einer derartigen Anordnung verhindert werden kann, vermeidet man ein Beschädigen der Befüllnadel durch ein Schrägstehen der pharmazeutischen Behältnisse oder medizinischen Packmittel. Des Weiteren kann man die Trägerplatte sicher aus einer Transport- und/oder Lagereinrichtung für pharmazeutische Behältnisse oder medizinischen Packmitteln entnehmen, beispielsweise durch Anheben. Des Weiteren ist es möglich Stopfen, die auf die jeweiligen pharmazeutischen Behältnisse aufgesetzt werden, in der richtigen Höhe zu positionieren und damit axiale Beweglichkeit zu vermeiden. Des Weiteren kann die axiale Beweglichkeit durch relativ einfache Maßnahmen behindert werden, die es nicht notwendig macht, zusätzliche Vorsprünge oder eine zusätzliche Platte vorzusehen, die einen Einlegeprozess beispielsweise der Trägerplatte in die Transport- und/oder Lagereinrichtung erschwert.

In einer Fortbildung der Erfindung ist in einer bevorzugten Ausführungsform vorgesehen, dass die Erhebungen bevorzugt knochenförmig mit einem Mittenabschnitt und zwei Seitenabschnitten ausgebildet ist. Durch die Schrägen und/oder Radien wird ein Verdrehen der eingelegten pharmazeutischen Behältnisse, wie oben beschrieben, verhindert.

Wenn die Erhebungen knochenförmig ausgebidet sind, weisen diese wenigstens im Bereich der Seitenabschnitte Schrägen und/oder Radien auf. Die Schrägen und/oder Radien ermöglichen es zudem, dass die pharmazeutischen Behältnisse oder medizinschen Packmittel, insbesondere Spritzen, Fläschchen oder Karpullen leichter eingelegt werden können. Des Weiteren ermöglichen die Einlaufschrägen bei pharmazeutischen Behältnissen oder medizinschen Packmitteln mit wenigstens einem nicht symmetrischen Flansch, z.B. einem Kragen eine Selbstzentrierung, insbesondere nach einem Hochhüpfen, d. h. einer axialen Bewegung. Auch sorgen sie für eine Selbstzentrierung bei einem Hochhüpfen über die Höhe der Erhebung bzw. einzelstehenden Bauteiles hinaus. Dies ist sogar möglich, wenn die pharmazeutischen Behältnisse um 15° verdreht sind.

Die Schrägen und/oder Radien werden bei knochenförmigen Erhebungen an den Seitenabschnitten ausgebildet. Alternativ zu den knochenförmigen Erhebungen sind einzelstehende Erhebungen, die ggf. ebenfalls Schrägen und/oder Radien aufweisen, möglich.

Bevorzugt sind jeder Durchgangsöffnung zwei Schrägen zugeordnet, entweder in Form von Seitenabschnitten bei einer knochenförmigen Erhebung oder bei einem einzelnstehenden Bauteil.

Zwar kann bei größeren, axialen Hüpfhöhen von beispielsweise mehr als 5 mm eine z. B. 5 mm hohe Erhöhung übersprungen werden und ein Heraushüpfen und Verdrehen des pharmazeutischen Behälters, insbesondere der Spritze, bevorzugt der Spritze mit einem Kragen, erfolgen. Die erfindungsgemäßen Einlaufschrägen sorgen dann dafür, dass ein automatisches Wiedereinlegen in die Normposition durch leichtes seitliches Rütteln erreicht wird, wobei der Selbstzentrierungseffekt auch bei verdreht liegenden Flanschen mit einem Drehwinkel von bis zu 15° greift.

In einer weitergebildeten Ausführungsform bildet die Trägerplatte ein Kastenprofil mit einer Oberseite und einer Unterseite aus. Ein derartiges Kastenprofil ist beispielsweise in der WO 2012/126582 gezeigt, deren Offenbarungsgehalt vollumfänglich in vorliegender Anmeldung mit aufgenommen wird.

Das Kastenprofil stellt eine hohe Stabilität der Trägerplatte zur Verfügung, da bei einem Kastenprofil eine hohe Biege- und Torsionssteifigkeit erreicht wird bei nur geringem Materialmehrverbrauch.

Bevorzugt weist das Kastenprofil eine Kastenprofilhöhe auf, die insbesondere im Bereich von 5 bis 50 mm, insbesondere von 10 bis 30 mm zwischen der Ober- und der Unterseite der Trägerplatte liegt. Derart ausgebildete Kastenprofile können gestapelt werden, bevorzugt mit einer Stapelhöhe von 15 bis 40 mm.

Besonders bevorzugt ist es, wenn die Trägerplatte Handhabungsbereiche umfasst. Die Handhabungsbereiche dienen zum Handling der Trägerplatte und ermöglichen es, die Trägerplatte beispielsweise aus Transport- und/oder Lagereinrichtungen für pharmazeutische Behältnisse zu entnehmen.

Die Steifigkeit des Kastenprofils wird noch erhöht, wenn Mittel zur Erhöhung der Steifigkeit auf der Zugseite der Trägerplatte angeordnet sind. Dies können beispielsweise miteinander verbindbare Rippen sein, die zwei benachbarte Durchgangsöffnungen miteinander verbinden. Diese Rippen können in einer vorteilhaften Ausgestaltung innerhalb einzelner Kastenprofilbereiche angeordnet sein.

In einer besonders bevorzugten Ausführungsform weist die Trägerplatte im Bereich der Durchgangsöffnung einen Wulst, bevorzugt einen umlaufenden Wulst, auf. Durch den Wulst, insbesondere den umlaufenden Wulst, wird die Kontaktfläche zwischen der Spritze und der Trägerplatte reduziert, da die Spritze nur auf dem Wulst aufliegt. Des Weiteren ermöglicht der Wulst es beispielsweise, die eingelegte Spritze als pharmazeutisches Behältnis mit Greifern aus der Trägerplatte zu heben.

Eine erfindungsgemäße Trägerplatte ist in besonders vorteilhafter Ausführung in einer Transport- und/oder Lagereinrichtung für pharmazeutische Behältnisse, insbesondere Spritzen, Fläschchen und Kapulen, einsetzbar. Diese umfasst einen Aufnahmebehälter, der die Trägerplatte im bestückten Zustand aufnimmt.

Um ein Hochhüpfen zu verhindern, kann eine Abdeckung des Aufnahmebehälters vorgesehen sein. Bevorzugt kann die Abdeckung in Form einer Folie, beispielsweise einer Siegelfolie, ausgeführt sein. Weitere Funktionselemente wie beispielsweise Dichteinrichtungen sind vorsehbar. Durch die erfindungsgemäße Ausbildung der Trägerplatte bleiben die in diese gelagerten Behältnisse hinsichtlich ihrer Lage zueinander beim Transport und der Lagerung auch über einen längeren Zeitraum in der Durchgangsöffnung zentriert und definiert ausgerichtet. Beschädigung der Behältnisse, die in Durchgangsöffnungen gelagert werden, können dadurch vermieden werden.

Die Erfindung soll nachfolgend beispielhaft anhand der Zeichnungen beschrieben werden.

Es zeigen:

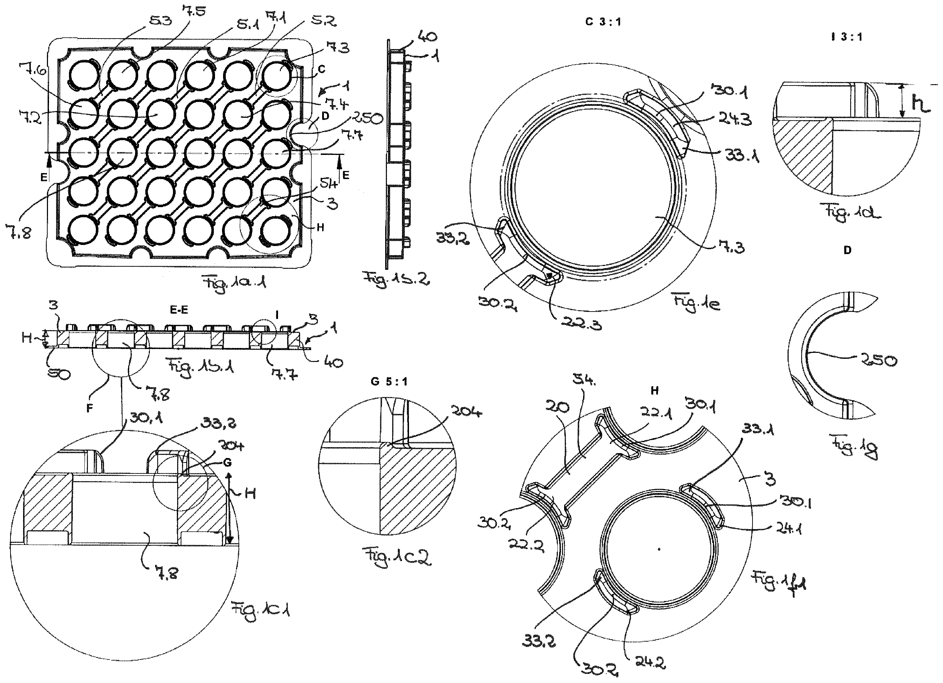

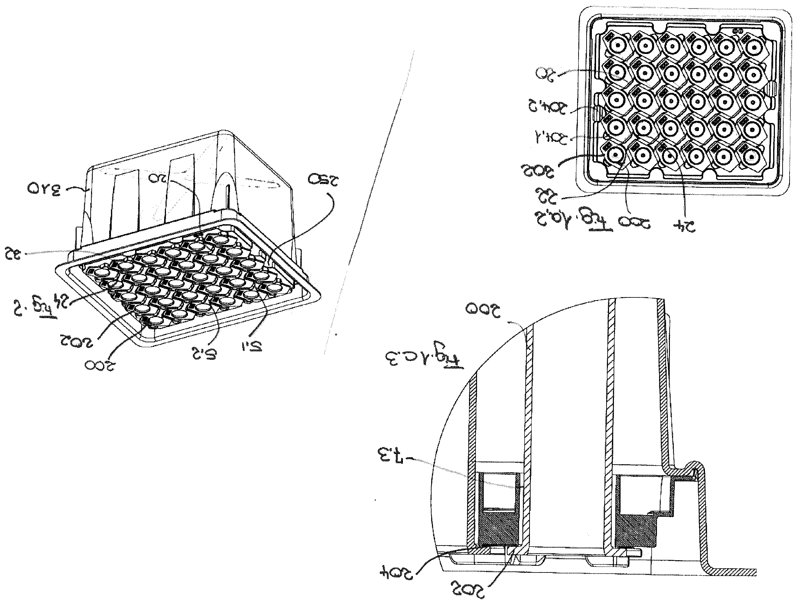

- Fig. 1a.1: eine Draufsicht auf eine erfindungsgemäße Trägerplatte ohne eingelegte pharmazeutische Behältnisse;
- Fig. 1a.2: eine Draufsicht auf eine erfindungsgemäße Trägerplatte mit eingelegten pharmazeutischen Behältnissen;
- Fig. 1b.1: eine Schnittansicht entlang der Linie E-E in Fig. 1a.1 der Trägerplatte, aus der das Kastenprofil hervorgeht;
- Fig. 1b.2: eine Seitenansicht der Trägerplatte gemäß Fig. 1a.1 aus der das Kastenprofil hervorgeht.
- Fig. 1c.1: Detail F aus Fig. 1b.1 umfassend eine Durchgangsöffnung ohne eingelegtes pharmazeutisches Behältnis;
- Fig. 1c.2: Detail G aus Fig.1c.1
- Fig. 1c.3: Durchgangsöffnung mit eingelegtem pharmazeutischen Behältnis
- Fig. 1d: Detail I aus Fig. 1b.1;
- Fig. 1e: Detail C aus Fig. 1a.1;

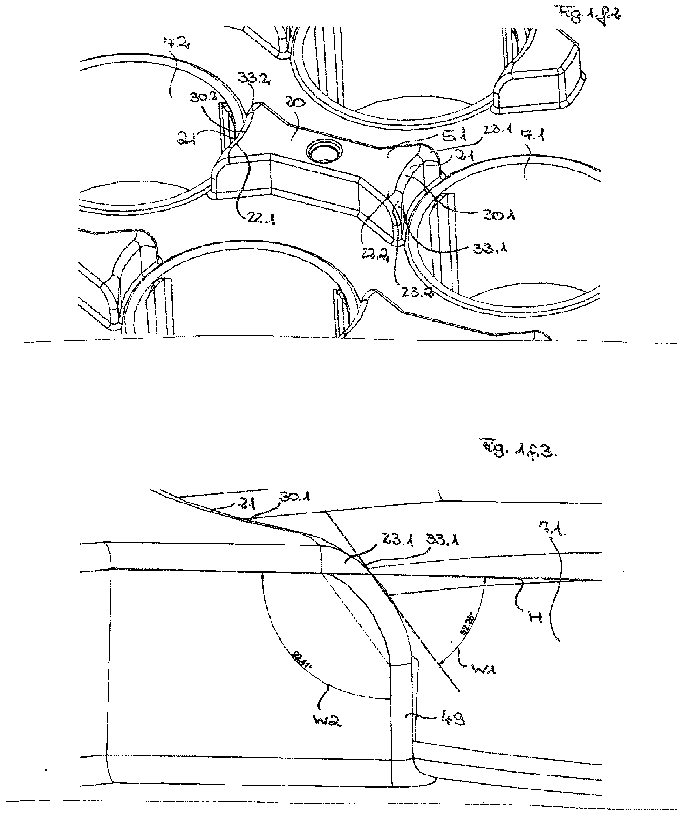

- Fig. 1f.1: Detail H aus Fig. 1a.1;
- Fig. 1f.2: detaillierte Ansicht der knochenförmigen Erhebung, die zwischen zwei Öffnungen ausgebildet wird
- Fig. 1f.3: Ansicht der Radien 33.1 der Erhebung
- Fig. 2: dreidimensionale Ansicht einer erfindungsgemäßen Trägerplatte eingesetzt in eine Transport- und/oder Lagereinrichtung ohne Abdeckung.

Fig.1a.1 zeigt eine Draufsicht auf eine erfindungsgemäße Trägerplatte ohne eingelegte Spritzen für pharmazeutische Behältnisse oder medizinischen Packmittel mit einem nicht zylindersymmetrischen Flansch, insbesondere Kragen. Die Trägerplatte 1 für pharmazeutische Behältnisse umfasst eine Oberseite 3, die vorliegend dargestellt ist. Auf die Oberseite 3 der Trägerplatte 1 sind Erhebungen 5.1, 5.2, 5.3, 5.4 aufgebracht, die zwischen den einzelnen Durchgangsöffnungen 7.1, 7.2, 7.3, 7.4, 7.5, 7.6, 7.7, 7.8 der Trägerplatte 1 angeordnet sind. Die Durchgangsöffnungen 7.7 und 7.8 liegen auf der Schnittlinie E-E in Fig. 1a.1 und werden daher auch in Fig. 1b.1, 1c.1 und 1c.2 dargestellt.

Die Erhebungen weisen eine Höhe (Fig. 1d) h auf, die bevorzugt im Bereich 2 mm bis 20 mm, insbesondere 4 mm bis 10 mm liegt. Die Mindesthöhe von 2 mm für die Erhebung bzw. das einzelstehende Bauteil ergibt sich aus der Höhe des nicht symmetrischen Flansches bzw. Kragens des pharmazeutischen Behältnisses, der bei etwa 2 mm liegt. Durch die Mindesthöhe wird sichergestellt, dass die Schrägen und/oder Radien auf den Flansch zur Zentrierung einwirken können, insbesondere bei einem Hochhüpfen. Die Obergrenze von 20 mm für die Erhebung folgt aus dem Eigengewicht der pharmazeutischen Behältnisse, das dann so schwer wird, dass ein Hochhüpfen nicht mehr möglich ist. Wie die Draufsicht auf die Oberfläche der Oberseite 3 der Trägerplatte 1 mit den einzelnen Erhebungen 5.1, 5.2, 5.3, 5.4 zeigt, ist die jeweilige Erhebung 5.1, 5.2, 5.3, 5.4 knochenförmig, wie in Fig. 1f.1 - 1f.3 detailliert dargestellt, d. h. sie umfasst einen Mittenabschnitt 20 sowie zwei Seitenabschnitte 22.1, 22.2. Die knochenförmige Erhebung ist beispielhaft, aber keineswegs zwingend. Die Höhen der Erhebung beträgt zwischen 2 und 20 mm. Des Weiteren umfasst die Erhebung erfindungsgemäß Schrägen 30.1, 30.2 und Radien 33.1, 33.2. Die Schrägen 30.1, 30.2 und Radien 33.1, 33.2 werden im Bereich der Seitenabschnitte 22.1, 22.2 der knochenförmigen Erhebung 5.1, 5.2, 5.3, 5.4 ausgebildet. Wie in Figur 1f.1 - 1f.3 gezeigt werden die Schrägen 30.1, 30.2 und Radien 33.1, 33.2 nicht nur in den Seitenabschnitten 22.1, 22.2 der knochenförmigen Erhebung ausgebildet, sondern können auch Teil einzelnstehender Bauteile 24.1, 24.2 auf der Oberseite 3 der Trägerplatte 1 sein. Bei den einzelnstehenden Bauteilen 24.1, 24.2 handelt es sich um Bauteile, die Schrägen 30.1, 30.2 und Radien 33.1, 33.2 analog zu den Schrägen und Radien der Seitenabschnitte 22.1, 22.2 der knochenförmigen Erhebungen aufweisen. Die Bauteile 24.1, 24.2 sind im Sinne der Erfindung Erhebungen und analog zu den Seitenabschnitten 22.1, 22.2 der knochenförmigen Erhebungen 20 ausgebildet. Die einzelstehenden Bauteile 24.1, 24.2 mit Schrägen 30.1, 30.2 und Radien 33.1, 33.2 werden bevorzugt im Randbereich der Oberfläche 3 ausgebildet in denen knochenförmige Erhebungen aus Platzgründen nicht angeordnet werden können. Sämtliche dargestellten Erhebungen weisen sowohl Schrägen als auch Radien auf. In der dargestellten Ausführungsform grenzen Schrägen und Radien direkt aneinander an. Der Effekt der Erfindung wird aber auch erreicht, wenn nur Schrägen oder nur Radien vorgesehen sind. Durch die Schrägen und/oder Radien wird erreicht, dass durch die Anschrägungen bzw. den Radius ein leichtes Einlegen der pharmazeutischen Behälter möglich ist. Des Weiteren ist hierdurch eine Selbstzentrierung auch bei um 15° verdreht eingelegtem pharmazeutischen Behältnis mit nicht symmetrischem Flansch, insbesondere Spritzenkörper mit einem Kragen möglich. Ein weiterer Vorteil der Schrägen und oder Radien ist, dass die pharmazeutischen Behältnisse, insbesondere die Spritzen, auch wenn sie sich in axialer Richtung über die Erhebung hinaus bewegen, z. B. Heraushüpfen, wieder in die Öffnung eingefädelt werden. Gleiche Bauteile wie in Fig. 1a1 sind mit gleichen Bezugsziffern belegt. Die Ausbildung der Schrägen 30.1, 30.2 und Radien 33.1, 33.2 ist detailliert in den Figuren 1f.2 und 1f.3 gezeigt. Die Radien sind verrundet, weisen aber einen mittleren Winkel gegenüber der Horizontalen H auf. Um für die erfindungsgemäße Selbstzentrierung zu sorgen ist es vorteilhaft, wenn der mittlere Winkel der Schrägen und/oder Radien gegenüber der Horizontalen H im Bereich 40° und 60°, bevorzugt zwischen 45° und 60° liegt. Um die Selbstzentrierung zu unterstützen kann darüber hinaus vorgesehen sein, dass die Schrägen und/oder Radien nicht unter 90° in die Öffnung gegenüber der Horizontalen H münden, sondern unter Winkeln größer als 90°, beispielsweise im Bereich von 91° bis 95°, bevorzugt 92° bis 93°. Der Einlauf der Spritzen in die Öffnung wird durch eine solche Maßnahme unterstützt.

In Fig. 1a.2 ist eine Ansicht der Trägerplatte 1 mit eingesetzten pharmazeutischen Behältnissen - hier Spritzenkörper 200 mit Spritzenkragen 202, gezeigt. Der Spritzenkragen ist eine spezielle Form eines nicht symmetrischen Flansches. Wie aus Fig. 1a.2 zu entnehmen ist, korrespondieren die Ränder 204.1, 204.2 der Spritzenkragen 202 sowohl mit Seitenabschnitten 22.1, 22.2 und dem Mittenabschnitt 20 der knochenförmigen Erhebung 5.1 ohne, dass sie an diesen im Normalfall anliegen. Durch die Schrägen 30.1, 30.2 und/oder Radien 33.1, 33.2 an den Seitenabschnitten der Erhebung wird der Spritzenkragen geführt und in die entsprechende Durchgangsöffnung (nicht gezeigt) geleitet. Die Schrägen 30.1, 30.2 dienen dazu, dass die pharmazeutischen Behältnisse leichter in die Öffnungen eingelegt werden können und bei einem Heraushüpfen in axialer Richtung wieder leicht in die Durchgangsöffnung eingefädelt werden können.

Fig. 1b.1 ist eine Ansicht entlang der Schnittlinie E-E in Fig. 1a.1. Deutlich zu erkennen ist die Oberseite 3 der Trägerplatte 1 sowie das Kastenprofil 40 der Trägerplatte 1. Das Kastenprofil 40 umfasst eine Höhe H, die bevorzugt im Bereich 5 bis 50 mm, bevorzugt 10 bis 30 mm, vorzugsweise bei 15 bis 20 mm, liegt. Die Höhe H bezeichnet die Höhe des Kastenprofils von der Oberseite 3 der Trägerplatte bis zur Unterseite 50 derselben. Des Weiteren sind in Fig. 1b.1 deutlich zu erkennen die Durchgangsöffnungen, in Fig. 1a.1 z. B mit 7.1, 7.2, 7.3 bezeichnet sind. Die Höhe h von mindestens 5 mm für das Kastenprofil stellt sicher, dass sich das Kastenprofil nicht verzieht. Ein Verzug ist problematisch, da dann ein sauberes Einfädeln in die Durchgangsöffnungen nicht mehr gewährleistet ist.

Fig. 1b.2 zeigt eine Seitenansicht des kastenförmigen Profils, der Trägerplatte 1, wobei die Durchgangsöffnungen nicht abgebildet sind.

In Fig. 1c.1 dargestellt ist das Detail F aus Fig. 1b.1. Deutlich zu erkennen ist die Durchgangsöffnung 7.8 sowie die Schräge 30.1 und der Radius, 33.2.

In Fig. 1c.2 ist das Detail G aus Fig. 1c.1 dargestellt. Gleiche Bauteile sind mit denselben Bezugsziffern gekennzeichnet. Deutlich in Fig. 1c.1 und insbesondere 1c.2 zu erkennen ist ein Wulst 204 auf dem im eingesetzten Zustand der Spritzenkragen (nicht dargestellt) eines Spritzenkörpers (nicht dargestellt) aufliegt.

In Fig. 1c.3 ist eine Durchgangsöffnung 7.3 mit eingesetztem pharmazeutischem Behältnis hier einer Spritze mit Spritzenkörper 200 und Spritzenkragen 202 gezeigt. Deutlich zu erkennen ist auch der Wulst 204, der bevorzugt umlaufend, auf der Oberseite der Trägerplatte ausgebildet ist. Der Spritzenkragen 202 der Spritze liegt auf dem Wulst 204 auf. Durch den Wulst 204 wird die Kontaktfläche zwischen der Spritze und der Trägerplatte reduziert. Des Weiteren kann die so gelagerte Spritze von Greifern aus der Trägerplatte gehoben werden.

Fig. 1d zeigt das Detail I aus Fig. 1b.1. Deutlich zu erkennen sind die Höhen h die Höhe der Erhebung, die im Bereich von 2 mm bis 20 mm, bevorzugt 4 mm bis 10 mm liegt. Die Höhe H des Kastenprofils ist in Fig. 1b.1 gezeigt und liegt bevorzugt im Bereich 10 mm bis 50 mm, insbesondere 10 mm bis 30 mm, bevorzugt 15 bis 20 mm. Bei der dargestellten Ausführungsform ist die Höhe h 5,5 mm, die Höhe H 15,5 mm. Die Höhe h der Erhebung verhindert ein Heraushüpfen in axialer Richtung, falls die Höhe der Bewegung in axialer Richtung geringer als die Höhe der Erhebung ist.

Die Fig. 1e ist das Detail C aus Fig. 1a.1, gezeigt. Zu sehen ist die Durchgangsöffnung 7.3 am Rand der Trägerplatte 1. Bei der Ausgestaltung gemäß Fig. 1e wird eine Schräge 30.1 und ein Radius 33.1 durch ein einzelstehendes Bauteil 24.3 zur Verfügung gestellt, die andere Schräge 30.2 und der andere Radius 33.2 durch den Seitenabschnitt 22.3 einer knochenförmigen Erhebung.

Wie sämtlichen zuvor dargestellten Durchgangsöffnungen 7.1, 7.2, 7.2, 7.4, 7.5, 7.6, 7.7, 7.8 sind der Durchgangsöffnung zwei Schrägen 30.1, 30.2, 31.1, 31.2 und Radien 33.1, 33.2, 35.1, 35.2 zugeordnet.

Fig. 1f.1 zeigt das Detail H aus Fig. 1a.1. Deutlich zu erkennen sind die Schrägen 30.1, 30.2 zum einen an den Seitenabschnitten 22.1, 22.2 der knochenförmigen Erhebung 5.4 und an den einzelstehenden Bauteilen 24.1, 24.2. Wiederum sind jeder Durchgangsöffnung genau zwei Schrägen und / oder Radien zugeordnet.

Fig. 1f.2 zeigt nochmals detailliert die knochenförmige Erhebung, die in Figur 1a.1 wie in Figur 1f.2 mit 5.1 bezeichnet ist. Die Öffnung zwischen denen die knochenförmige Erhebung 5.1 angeordnet ist, ist wie in Figur 1a.1 mit 7.1 bzw. 7.2 bezeichnet. Die knochenförmige Erhebung 5.1 weist zur Unterstützung der Zentrierung von in die Öffnungen 7.1, 7.2 eingeführten pharmazeutischen Behältnissen, bevorzugt solchen mit einem Flansch einen der Öffnung 7.1, 7.2, gegenüberliegenden mittleren Bereich 21 im Bereich der Seitenabschnitte 22.1, 22.2, auf. Desweiteren schließen sich an den mittleren Bereich 21 je zwei Seitenbereich 23.1, 23.2 an. Der mittlere Bereich 21 umfasst eine Schräge 30.1, 30.2 und die Seitenbereich 23.1, 23.3, Radien 33.1, 33.2. Wie im Detail in Figur 1f.3 gezeigt, weisen die Schrägen 30.1, 30.2 und die Radien 33.1, 33.2, gegenüber einer Horizontalen H einen mittleren Winkel W1 auf um den der Radius 33.1, 33.2 und/oder die Schräge 30.1, 30.2, geneigt ist. Bevorzugt liegt der mittlere Winkel W1 mit dem die Schräge 30.1, 30.2 und/oder der Radius geneigt ist im Bereich 40° bis 65°, bevorzugt 45° bis 60°. In der dargestellten Ausführungsform liegt er bei 52, 25°. Wird der Winkle W1 in dem angegebenen Bereich gewählt, so erfolgt eine Selbstzentrierung von in die Öffnung 7.1 eingestellten pharmazeutischen Behältnissen, auch dann wenn die Behältnisse, bevorzugt mit einem Flansch schräg in die Öffnung eingebracht werden oder aus dieser heraushüpfen. Dies wird dadurch erreicht, dass die Radien und/oder Schrägen unter einem Winkel W1 gegenüber der Horizontalen geneigt sind und ein Ausrichten der Behältnisse unterstützen. Besonders bevorzugt ist es wie in Fig. 1f.3 gezeigt, wenn die Öffnungen 7.1, 7.2 nicht einen 90°-Winkel zur Horizontalen H aufweisen, sondern Winkel W2 größer als 90°, bevorzugt im Bereich 91° bis 95°. Dann sind die Öffnungen im unteren Abschnitt 49 schräg ausgebildet, was widerum das Ausrichten von schräg eingebrachten Behältnissen unterstützt.

Fig. 1g zeigt das Detail D aus Figur 1a.1. In Fig. 1g ist der Handhabungsbereich 250 gezeigt mit dem die Trägerplatte 1 beispielsweise aus einer Wanne 310 wie in Fig. 2 dargestellt herausgehoben werden kann.

In Fig. 2 sind eine Vielzahl von pharmazeutischen Behältnissen oder medizinischen Packmitteln mit einem nicht symmetrischen Flansch, insbesondere einem Kragen 202 eingesetzt in eine erfindungsgemäße Trägerplatte 1, die wiederum eingesetzt ist in eine Transport- oder Lagereinrichtung, die als Wanne 310 ausgebildet ist, gezeigt. In die Transport- und Lagervorrichtung kann die mit den pharmazeutischen Behältnissen, vorliegend den Spritzenkörpern 200 mit Kragen 202 bestückte Trägerplatte 1 eingebracht werden. Der Aufnahmebehälter 310 ist bevorzugt wannenförmig ausgebildet. Die Trägerplatte wird in den wannenförmigen Aufnahmebehälter 310 bevorzugt eingehängt. Die Lagerung der Trägerplatte 1 im Aufnahmebehälter kann durch Kraft- und/oder Formschluss realisiert werden. Wie aus Fig. 2 deutlich hervorgeht, sind auf der Oberfläche H des Trägerelements eine Vielzahl von Erhebungen 5.1, 5.2 vorgesehen, die zumindest teilweise knochenförmig ausgebildet sind, d. h. mit einem Mittenabschnitt 20 und zwei Seitenabschnitten 22.1. 22.2. Wie aus Fig. 2 deutlich zu ersehen ist, korrespondiert der Kragen 202 der jeweiligen pharmazeutischen Behältnisse mit den Seitenflächen der Seitenabschnitte 22.1, 22.2 der Erhebungen ohne im Normalfall an diesen anzustoßen. Durch die Ausgestaltung der Erhebungen mit einer Höhe h im Bereich von 2 mm bis 20 mm, bevorzugt 4 mm bis 10 mm, mit Schrägen und / oder Radien wird das Einlegen der Spritzen unterstützt. Des Weiteren wird bei einer axialen Bewegung der Spritzen über die Erhebung hinaus ein Verdrehen der Behältnisse und Überlagern der einzelnen Kragen weitgehend verhindert.

Zum Transport kann die Wanne mit einer Abdeckung, beispielsweise einer Folie (nicht gezeigt) versehen sein. In einem solchen Fall wird durch die Abdeckung eine Bewegung der Spritzen über die Erhebung hinaus in axialer Richtung vollständig verhindert, da in einem solchen Fall die Spritzen an der Abdeckung anschlagen würden.

Des Weiteren wird eine Trägerplatte angegeben, die eine Verdrehsicherung zur Verfügung stellt, wobei die Verdrehsicherung ein Verdrehen der einzelnen in die Trägerplatte eingelegten pharmazeutischen Behälter bei axialer Bewegung verhindert.

## Patentansprüche

1. Trägerplatte für pharmazeutische Behältnisse oder medizinische Packmittel mit einem nicht zylindersymmetrischen Flansch, bevorzugt Kragen (202) insbesondere Spritzen, Fläschchen oder Karpulen, umfassend eine Mehrzahl von Durchgangsöffnungen (7.1, 7.2, 7.3) zur Aufnahme der pharmazeutischen Behältnisse oder medizinischer Packmittel,
wobei die Trägerplatte wenigstens eine über eine Oberseite der Trägerplatte hinausragende Erhebung (5.1, 5.2, 5.3) und / oder einzelstehende Bauteile ( 24.1, 24.2, 24.3) mit einer Höhe (h) und wenigstens einer Schräge (30.1, 30.2) und / oder einem Radius (33.1, 33.2) umfasst, wobei jeder der Mehrzahl von Durchgangsöffnungen (7.1, 7.2, 7.3) wenigstens eine Erhebung und / oder ein Bauteil zugeordnet ist, **dadurch gekennzeichnet, dass** die Erhebungen und / oder das Bauteil derart ausgestaltet ist, dass wenigstens eine Schräge (30.1, 30.2) und / oder Radius (33.1, 33.2) mit dem nicht zylindersymmetrischen Flansch (202) zusammenwirkt, so dass die pharmazeutischen Behältnisse bei Winkelabweichungen von Winkeln > 0° bis ungefähr 20° ausgerichtet werden und ein Verdrehen vermieden wird.

2. Trägerplatte nach Anspruch 1,
**dadurch gekennzeichnet, dass**,
die Schräge (30.1, 30.2) und/oder der Radius (33.1, 33.2) einen mittleren Winkel W 1 gegenüber der Horizontalen (H), bevorzugt im Bereich 40° bis 65°,
insbesondere 45° bis 60° aufweist.

3. Trägerplatte nach einem der Ansprüche 1 bis 2,
**dadurch gekennzeichnet, dass**
die Durchgangsöffnung (7.1, 7.2, 7.3) einen Winkel W 2 größer als 90°, insbesondere im Bereich 91° bis 95° gegenüber der Horizontalen aufweist.

4. Trägerplatte nach einem der Ansprüche 1 bis 3,
**dadurch gekennzeichnet, dass**,
die Trägerplatte derart ausgebildet ist, dass jeder Durchgangsöffnung zwei Erhebungen mit Schrägen (30.1, 30.2) und / oder Radien (33.1, 33.2) und / oder einzelstehende Bauteile zugeordnet sind.

5. Trägerplatte nach einem der Ansprüche 1 bis 4,
**dadurch gekennzeichnet, dass**
die Schrägen (30.1, 30.2) und / oder Radien (33.1, 33.2) an dem einzelstehenden Bauteil (24.1, 24.2, 24.3) und/oder an einem Seitenabschnitt (22.1, 22.2, 22.3) einer Erhebung 5.1, 5.2, 5.3 ausgebildet ist.

6. Trägerplatte nach einem der Ansprüche 1 bis 5,
**dadurch gekennzeichnet, dass**
die Höhe (h) der Erhebung und / oder des einzelstehenden Bauteils mindestens 2 mm, bevorzugt 2 mm bis 20 mm, insbesondere 4 mm bis 10 mm, beträgt.

7. Trägerplatte nach einem der Ansprüche 1 bis 6,
**dadurch gekennzeichnet, dass**
die Erhebung einen Mittenabschnitt (20) und zwei Seitenabschnitten (22.1, 22.2) umfasst.

8. Trägerplatte nach einem der Ansprüche 1 bis 7,
**dadurch gekennzeichnet, dass**
die Erhebung und/oder das einzelstehende Bauteil mit Schräge (30.1, 30.2) und/oder Radien (33.1, 33.2) derart ausgestaltet ist, dass in die Durchgangsöffnung (7.1, 7.2, 7.3, 7.4, 7.5, 7.6, 7.7, 7.8) eingebrachte pharmazeutischen Behältnisse mit nicht symmetrischem Flansch verdrehsicher gehalten werden .

9. Trägerplatte nach einem der Ansprüche 1 bis 8,
**dadurch gekennzeichnet, dass**
die Trägerplatte ein Kastenprofil, das die Oberseite und eine Unterseite umfasst, und
das Kastenprofil eine Kastenprofilhöhe (H), bevorzugt im Bereich 5 mm bis 50 mm, insbesondere 10 mm bis 30 mm zwischen der Oberseite (42) und der Unterseite (44) der Trägerplatte aufweist.

10. Trägerplatte nach einem der Ansprüche 1 bis 9,
**dadurch gekennzeichnet, dass**
die Trägerplatte stapelbar ist, bevorzugt mit einer Stapelhöhe von 14 mm bis 40 mm.

11. Trägerplatte nach einem der Ansprüche 1 bis 10,
**dadurch gekennzeichnet, dass**
die Trägerplatte Handhabungsbereiche (250) umfasst.

12. Trägerplatte nach einem der Ansprüche 1 bis 11,
**dadurch gekennzeichnet, dass**
die Trägerplatte Mittel zur Erhöhung der Steifheit umfasst.

13. Trägerplatte nach einem der Ansprüche 1 bis 12,
**dadurch gekennzeichnet, dass**
die Trägerplatte im Bereich einer Durchgangsöffnung einen Wulst (204), bevorzugt einen umlaufenden Wulst, umfasst.

14. Transport- und/oder Lagereinrichtung für pharmazeutische Behältnisse oder medizinische Packmittel, insbesondere Spritzen, Fläschchen und Karpullen, mit einem nicht symmetrischen Flansch, bevorzugt Kragen umfassend eine Trägerplatte gemäß einem der Ansprüche 1 bis 13, die von einem Aufnahmebehälter bevorzugt einer Wanne 310 aufgenommen wird und die Transport- und/oder Lagereinrichtung zumindest eine den Aufnahmebehälter verschließende Abdeckung aufweist.

## Claims

1. A carrier plate for pharmaceutical containers or medicinal packaging materials having a non-cylinder-symmetrical flange, preferably a collar (202), in particular syringes, ampoules, or cartridges, comprising a plurality of passage openings (7.1, 7.2, 7.3) for accommodating the pharmaceutical containers or medicinal packaging materials,
wherein the carrier plate comprises at least one protrusion (5.1, 5.2, 5.3) protruding beyond an upper side of the carrier plate and/or isolated components (24.1, 24.2, 24.3) having a height (h) and at least one bevel (30.1, 30.2) and/or one radius (33.1, 33.2), wherein at least one protrusion and/or one component is associated with each of the plurality of passage openings (7.1, 7.2, 7.3), **characterized in that** the protrusions and/or the component are designed such that at least one bevel (30.1, 30.2) and/or radius (33.1, 33.2) interacts with the non-cylinder-symmetrical flange (202), and therefore the pharmaceutical containers are aligned in the event of angle deviations from angles > 0° to approximately 20° and twisting is avoided.

2. The carrier plate according to Claim 1,
**characterized in that** the bevel (30.1, 30.2) and/or the radius (33.1, 33.2) has a mean angle W 1 in relation to the horizontal (H), preferably in the range of 40° to 65°, in particular 45° to 60°.

3. The carrier plate according to any one of Claims 1 to 2,
**characterized in that** the passage opening (7.1, 7.2, 7.3) has an angle W 2 greater than 90°, in particular in the range of 91° to 95°, in relation to the horizontal.

4. The carrier plate according to any one of Claims 1 to 3,
**characterized in that** the carrier plate is formed such that two protrusions having bevels (30.1, 30.2) and/or radii (33.1, 33.2) and/or isolated components are associated with each passage opening.

5. The carrier plate according to any one of Claims 1 to 4,
**characterized in that** the bevels (30.1, 30.2) and/or radii (33.1, 33.2) are formed on the isolated component (24.1, 24.2, 24.3) and/or on a lateral section (22.1, 22.2, 22.3) of a protrusion 5.1, 5.2, 5.3.

6. The carrier plate according to any one of Claims 1 to 5,
**characterized in that** the height (h) of the protrusion and/or the isolated component is at least 2 mm, preferably 2 mm to 20 mm, in particular 4 mm to 10 mm.

7. The carrier plate according to any one of Claims 1 to 6,
**characterized in that** the protrusion comprises a middle section (20) and two lateral sections (22.1, 22.2).

8. The carrier plate according to any one of Claims 1 to 7,
**characterized in that** the protrusion and/or the isolated component having bevel (30.1, 30.2) and/or radii (33.1, 33.2) is designed such that pharmaceutical containers, which are introduced into the passage opening (7.1, 7.2, 7.3, 7.4, 7.5, 7.6, 7.7, 7.8), having non-symmetrical flange are held twist-locked.

9. The carrier plate according to any one of Claims 1 to 8,
**characterized in that** the carrier plate has a box profile, which comprises the upper side and a lower side, and
the box profile has a box profile height (H), preferably in the range of 5 mm to 50 mm, in particular 10 mm to 30 mm between the upper side (42) and the lower side (44) of the carrier plate.

10. The carrier plate according to any one of Claims 1 to 9,
**characterized in that** the carrier plate is stackable, preferably with a stack height of 14 mm to 40 mm.

11. The carrier plate according to any one of Claims 1 to 10,
**characterized in that** the carrier plate comprises handling regions (250).

12. The carrier plate according to any one of Claims 1 to 11,
**characterized in that** the carrier plate comprises means for enhancing the rigidity.

13. The carrier plate according to any one of Claims 1 to 12,
**characterized in that** the carrier plate comprises a bead (204), preferably a circumferential bead, in the region of a passage opening.

14. A transportation and/or storage unit for pharmaceutical containers or medical packaging material, in particular syringes, ampoules, and cartridges, having a nonsymmetrical flange, preferably a collar, comprising a carrier plate according to any one of Claims 1 to 13, which is accommodated by a receptacle container, preferably a trough 310 and the transportation and/or storage unit has at least one cover which closes the receptacle container.

## Revendications

1. Plaque support pour récipients pharmaceutiques ou emballages médicaux ayant une bride, de préférence une collerette (202), non cylindrique symétrique, en particulier des seringues, flacons ou cartouches, présentant une pluralité d'ouvertures traversantes (7.1, 7.2, 7.3) pour recevoir les récipients pharmaceutiques ou les emballages médicaux, la plaque support présentant au moins une élévation (5.1, 5.2, 5.3) dépassant d'une face supérieure de la plaque support et/ou des éléments individuels (24.1, 24.2, 24.3) avec une hauteur (h) et au moins un chanfrein (30.1, 30.2) et/ou un rayon (33.1, 33.2), au moins une élévation et/ou un élément étant associés à chacune de la pluralité d'ouvertures traversantes (7.1, 7.2, 7.3), **caractérisée en ce que** les élévations et/ou l'élément sont conçus de telle sorte qu'au moins un chanfrein (30.1, 30.2) et/ou un rayon (33.1, 33.2) coopère avec la bride non cylindrique symétrique (202) de façon que les récipients pharmaceutiques soient alignés en cas d'écarts angulaires d'angles > 0° jusqu'à environ 20° et qu'une rotation soit évitée.

2. Plaque support selon la revendication 1,
**caractérisée en ce que**,
le chanfrein (30.1, 30.2) et/ou le rayon (33.1, 33.2) présentent un angle moyen W1 par rapport à l'horizontale (H), de préférence dans la plage de 40° à 65°, en particulier de 45° à 60°.

3. Plaque support selon l'une des revendications 1 à 2,
**caractérisée en ce que**
l'ouverture traversante (7.1, 7.2, 7.3) présente un angle W2 supérieur à 90°, en particulier dans la plage de 91° à 95°, par rapport à l'horizontale.

4. Plaque support selon l'une des revendications 1 à 3, **caractérisée en ce que** la plaque support est conçue de telle sorte que deux élévations avec des chanfreins (30.1, 30.2) et/ou des rayons (33.1, 33.2) et/ou des éléments individuels sont associés à chaque ouverture traversante.

5. Plaque support selon l'une des revendications 1 à 4, **caractérisée en ce que** les chanfreins (30.1, 30.2) et/ou les rayons (33.1, 33.2) sont formés sur l'élément individuel (24.1, 24.2, 24.3) et/ou sur une partie latérale (22.1, 22.2, 22.3) d'une élévation 5.1, 5.2, 5.3.

6. Plaque support selon l'une des revendications 1 à 5, **caractérisée en ce que** la hauteur (h) de l'élévation et/ou de l'élément individuel est d'au moins 2 mm, de préférence de 2 mm à 20 mm, en particulier de 4 mm à 10 mm.

7. Plaque support selon l'une des revendications 1 à 6, **caractérisée en ce que** l'élévation présente une partie centrale (20) et deux parties latérales (22.1, 22.2).

8. Plaque support selon l'une des revendications 1 à 7, **caractérisée en ce que** l'élévation et/ou l'élément individuel avec un chanfrein (30.1, 30.2) et/ou des rayons (33.1, 33.2) est conçu(e) de telle sorte que les récipients pharmaceutiques à bride non symétrique introduits dans l'ouverture traversante (7.1, 7.2, 7.3, 7.4, 7.5, 7.6, 7.7, 7.8) sont maintenus de manière à exclure toute rotation.

9. Plaque support selon l'une des revendications 1 à 8, **caractérisée en ce que** la plaque support forme un profilé en caisson qui présente la face supérieure et une face inférieure, et
le profilé en caisson présente une hauteur de profilé en caisson (H), de préférence dans la plage de 5 mm à 50 mm, en particulier de 10 mm à 30 mm, entre la face supérieure (42) et la face inférieure (44) de la plaque support.

10. Plaque support selon l'une des revendications 1 à 9, **caractérisée en ce que** la plaque support est empilable, de préférence avec une hauteur d'empilage de 14 mm à 40 mm.

11. Plaque support selon l'une des revendications 1 à 10, **caractérisée en ce que** la plaque support présente des zones de manipulation (250).

12. Plaque support selon l'une des revendications 1 à 11, **caractérisée en ce que** la plaque support présente des moyens pour augmenter la rigidité.

13. Plaque support selon l'une des revendications 1 à 12, **caractérisée en ce que** la plaque support présente un bourrelet (204), de préférence un bourrelet périphérique, dans la zone d'une ouverture traversante.

14. Dispositif de transport et/ou de stockage pour récipients pharmaceutiques ou emballages médicaux, en particulier des seringues, flacons et cartouches, ayant une bride, de préférence une collerette, non symétrique, comprenant une plaque support selon l'une des revendications 1 à 13 qui est reçue par un récipient de réception, de préférence un bac 310, et le dispositif de transport et/ou de stockage présente au moins un couvercle fermant le récipient de réception.
